# EUROPÄISCHE PATENTANMELDUNG

(11) **EP 1 213 016 A2**
(43) Veröffentlichungstag der Anmeldung: **12.06.2002**
(21) Anmeldenummer: 01126608.7
(22) Anmeldetag: 07.11.2001
(51) Int. Cl.: A61K 9/72, A61K 9/70, A61L 9/00

(54) **Chip-Systeme zur kontrollierten Emission chemosensorisch wirkender Stoffe**

(30) Priorität: 08.12.2000 DE 10061057
(71) Anmelder: Liedtke, Rainer K., Dr., 82027 Grünwald (DE)
(72) Erfinder: Liedtke, Rainer K., Dr., 82027 Grünwald (DE)
(74) Vertreter: Leson, Thomas Johannes Alois, Dipl.-Ing.

(57) **Zusammenfassung**

Die Erfindung betrifft Verfahren und Vorrichtungen von Chip-Systemen zur kontrollierten Emission chemosensorisch wirkender Stoffe, insbesondere zur Erzeugung biologischchemosensorischer Reaktionen. Die Chip-Systeme emittieren kontrolliert wie auch programmierbar Stoffe, die an chemosensorischen Rezeptoren biochemisch Transduktionen für neuronal vermittelte Signale an spezifische Strukturen des Zentralnervensystems auslösen. Das neue Verfahren und seine Vorrichtungen ermöglichen für chemosensorisch aktive Stoffe zahlreiche neue Anwendungen in medizinischen und biologischen Bereichen.

## Beschreibung

Die Erfindung betrifft Verfahren und Vorrichtungen zu Chip-Systemen zur kontrollierten Emission chemosensorisch wirkender Stoffe, insbesondere zur Erzeugung biologisch chemosensorischer Reaktionen.

Es ist bekannt, daß im biologischen Bereich zahlreiche Pflanzen Naturstoffe als Signale emittieren, die biologische Reaktionen bei Insekten erzeugen, die entsprechende Biosensoren, chemosensorische Antennenorgane, für diese Signale haben. Ebenso können Insekten auch entsprechende chemische Signalstoffe in die Umwelt emittieren und hierüber beispielsweise zur Nahrungsssuche oder zur Reproduktion kommunizieren.

Bekannt ist, daß auch im Humanbereich sowie im Säugetierbereich mit Hilfe chemosensorisch geruchsaktiver Stoffe erhebliche Einflüsse auf Körperfunktionen, insbesondere im emotionalen Bereich, erzeugt werden können. Hierbei ist der erste biologische Anlandungspunkt für solche chemosensorisch wirkenden Reize die sogenannte Regio Olfactoria des Riechsinnesapparates, ein anatomisches Gebiet das im oberen hinteren Bereich der dritten nasalen Muschel in der Nasenhöhle liegt. Dessen zur Luftbahn der Nasenhöhle gewandte Oberfläche besteht aus primären Sinneszeilen, die ein Feld von Chemorezeptoren repräsentieren an denen eine sogenannte Transduktion, eine biochemische Wandlung der Rezeptoren-Reaktion in Auslösung elektrischer Potentiale erfolgt. Verarbeitungszentrum des zentralen Nervensystems für die von dort, über die Umschaltstation des Bulbus Olfactorius zwischenverarbeiteten, Reizsignale ist das Limbische System. Dieses spielt im Wechselspiel mit einigen anderen Strukturen, insbesondere dem Hypothalamus und cortikalen Projektionen, eine koordinative, wie auch integrierende Rolle eines "emotionalen Gehirns",

Im Rahmen verschiedener wissenschaftlicher biologischer und medizinischer Untersuchungen mit unterschiedlichen transnasal inhalierten Geruchsstoffen, meist solchen natürlichen Ursprungs, liessen sich sowohl Stimmungsänderungen wie auch Veränderungen im EEG nachweisen (Diego MA et al., Int. J. Neurosci. 1998 96 (3/4) 217), wobei die EEG Änderungen auch Bezüge zur Art der Gerüche auswiesen (Lee, CF et al., Ann. Physiol Anthrop. 1994 13 (5) 281; Harada, H. et al., Clin. Electroencephalogr. 1998 29 (2) 96). Neurophysiologische Effekte konnten auch bei Ratten im Riechhirn festgestellt werden (Zibrowski, E.M et al, Brain Res. 1998 800 (2) 207). Mehr spezifisch und ebenfalls nur beispielsweise sei noch auf Essverhalten und Reproduktion eingegangen. So wurde festgestellt, daß übergewichtige Frauen auf die Exposition von Nahrungsgerüchen mit einem anderen Muster der Speichelbildung reagieren, als dies bei normalgewichtigen Frauen der Fall war (Epstein LH et al, Psychosom. Med. 1996, 58 (2) 160). Bei Primaten liessen sich dabei Interaktionen des orbitofrontalen Cortex mit elektrophysiologischen Reaktionen olfaktorischer Neurone auf Essgerüche nachweisen (Critchley HD et. al J. Neurophysiol, 1996 (75(4) 1673); O' Doherty J. et al, Neuroreport 2000, 11(2) 399), auch, daß unter anderem olfaktorische Stimuli als Signale auf Nahrung erscheinen, diese sogar schon begannen bevor Nahrungsaufnahme eintrat (Bray GA, Proc. Nutr. Soc., 2000, 59(3) 373); Weibliche Mäuse die kopuliert haben und hiernach dem Geruch eines fremden Männchens ausgesetzt werden, unterliegen hierdurch solchen hormonellen Änderungen, die in einer Blockade ihrer Trächtigkeit (pregnancy block) resultieren (Kaba, H, et al., Neuroscience 1988, 25(3) 1007; Li, CS. et al, Neurosci. Letter 1994, 176 (1) 5;, Kaba H. et al, Science 1994, 265(5169) 262).

Daß zentrale Effekte auch nicht auf Stoffe begrenzt sind, die gemeinhin in der Pharmakologie als "Geruchsstoffe" angesehen werden, scheint auch für zahlreiche andere Stoffe zu gelten. So löste beispielsweise das Neuropeptid Oxytocin, primär angesehen als eine Art von Uterus- und Milchdrüsen-spezifischem Hormon, bei Auftragung auf die Regio Olfactoria, überraschenderweise bei Mäusen und Ratten schnelle zentral angstlösende, anti-depressive und antiagressive Effekte aus. Dabei wurde auch die Auslösung afferenter Nervensignale zu bestimmten involvierten Hypothalamuskernen nachgewiesen (I.D. Neuman, et al, Abstract XXXIst. Congress der Internat. Society of Psychoneuroendocriniology (ISPNE) Melbourne, Oct. 2000).

Aus diesen aufgeführten Beispielen ergibt sich, daß somit über eine olfaktorische Route pharmakodynamisch tiefergreifende Effekte auslösbar sind.

Um chemosensorisch wirkende Stoffe aber geeignet zur Auslösung von Bioreaktionen einsetzen zu können sowie die biologischen Effekte herbeizuführen, insbesondere für therapeutische oder diagnostische Anwendungen, erfordert es, daß die Stoffe auch in einer geeignet kontrollierten Form emittiert werden. Dies ist mit bisher bekannten Vorrichtungen aber nicht erreichbar.

Der Zugang zur sensorischen Regio Olfactoria ist durch die anatomischen Bedingungen dieser spezifischen Region bestimmt, insbesondere aber auch dadurch, daß es sich hier um Chemorezeptoren an Nervenstrukturen handelt, was auch die hohe Sensitivität dieser Region erklärt und woraus sich auch die nur geringe Dosiserfordernis zur Auslösung von Effekten ableitet. Weiterhin durch die Tatsache, daß es sich um eine anatomisch kleine und verborgen lokalisierte Fläche handelt, die nur über Luftkanäle erreicht werden kann. Eine Applikationsform, mit der ein Stoff diese Region erreichen kann erfordert somit, entsprechend der biologisch natürlichen Funktionen dieser Region, eine geeignet flüchtige Form, technisch somit Formulierungen mit in Gasen feinst verteilt schwebenden Teilchen, mit denen die chemosensorisch aktiven Stoffe zu diesem spezifischen Rezeptorareal transportiert werden.

Ein technische Möglichkeit wären Formulierungen, die herkömmlich als Nasensprays bezeichnet werden. Bei den derzeit verfügbaren konventionellen Nasensprays erreicht aber beispielsweise der quantitativ weit überwiegende Anteil, der in diesen

Formulierungen enthaltenen Wirkstoffe nur den resorptiven Schleimhautbereich im Bereich der unteren Nasenhöhle. Dies ist in diesem Falle auch pharmazeutischtechnisch beabsichtigt, da die pharmakokinetische Funktion von Nasensprays, als eine der Varianten systemischer Verabreichungen, den Stofftransport über die unspezifische Nasenschleimhaut in das Blut fördern sollen, oder im anderen Falle eine lokale Absetzung der Stoffe bedingen soll, um dort topische Wirkungen zu erzeugen. Demgegenüber besitzt aber die, topographisch höher liegende Regio Olfactoria, als chemosensorisches Nervenorgan. nicht über ein resorptives Epithel, sondern hat sogar diesem entgegengesetzt eine sekretorische Funktion.

Bekannt sind auch sogenannte Riechstreifen, wie sie beispielsweise bei Parfümerien oder Parfüm-Entwicklern für Geruchstests eingesetzt sind. Dort werden bestimmte Riechstoffe auf saugfähige Papierstreifen getropft und diese dann zwecks grober qualitativer Geruchsprobe an die Nase gehalten. Solcherart Hilfsmittel erlauben aber weder eine exakte Stoffdosierung noch eine reproduzierbar kontrollierte Evaporation. Sie zeigen sie unter anderem sofortige Verluste durch unkontrollierte Verdunstung und sind nicht lagerfähig. Es ist bekannt, daß UV Licht viele, wenn nicht gar die meisten, Geruchsstoffe zerstört. Daher sind Methoden wie das einfache ungeschützte Aufbringen von Stoffen auf Papiere oder Folien ungeeignet. Geringfügig besser lagerfähig ist eine verbesserte Form solcher Riechpapiere, die ebenfalls als Geruchsmuster eingesetzt werden, und bei denen die Riechstoffe vor ihrem Auftragen auf das Papier erst in kleine kapselartige Partikel gefüllt sind. Die Hülle der Partikel reisst dann bei äusserer mechanischer Druckeinwirkung auf. Hiernach entweichen aber auch in diesem Falle dann die darin eingeschlossene Geruchskomponenten insgesamt und unkontrolliert, sind dann auch zusätzlich wieder thermischen Einflüssen und Lichteinflüssen ausgesetzt.

Keiner dieser bisherigen Ansätze genügt daher den technischen Erfordernissen, die an ein kontrolliertes System angelegt werden müssen oder die den Erfordernissen therapeutischer, diagnostischer oder biologisch kontrollierter Anwendungen entspricht.

Der Erfindung liegt die Aufgabe zugrunde, eine kontrollierte Emission chemosensorisch wirkender Stoffe zu erreichen, insbesondere zur Erzeugung biologisch chemosensorischer Reaktionen.

Diese Aufgabe wird dadurch gelöst, daß Chip-Systeme zur kontrollierten Emission chemosensorischer Stoffe eingesetzt werden und wobei die Chips-Systeme als Mehrkomponentensysteme so zusammengesetzt sind, daß die chemosensorisch wirkenden Stoffe in zweifacher Weise kontrolliert als flüchtige Gemische freigesetzt werden, wobei die thermodyamische Diffusionsaktivität der Stoffe durch als Emissions-Promoter dienende Hilfsstoffe und die Emissionsrate der Stoffe durch eine als Diffusionskontrolle dienende Schicht kontrolliert wird, und wobei die Promotion chemisch, physikalisch oder biologisch geregelt werden kann, und wobei sich eine diesem entsprechende Vorrichtung zusammensetzt aus einer Trägerschicht mit den chemosensorisch aktiven Stoffen, einer die Promoter enthaltenden Schicht, die mit weiteren Schichten, die Hilfsvorrichtungen für die Promotion enthalten, verbunden sein kann, einer Emissions-Kontrollschicht, die als Membran oder Polymermatrix ausgebildet ist, sowie einem alle Komponenten einbeziehenden Träger an dessen äusserer Unterseite sich eine Haftschicht zur Fixierung des Systems und an dessen Oberseite sich eine von der Emissions-Kontrollschicht entfernbare Deckschicht befinden kann.

In einer weiteren Ausbildung der Erfindung erfolgt, um den praktischen Einsatz zu verbessern und auszuweiten, die Kontrolle der als Promoter dienenden Hilfsstoffe mechanisch, thermisch, elektrisch, magnetisch, biologisch, chemisch oder biochemisch, oder als eine Kombination solcher Massnahmen, wobei die Regelung der Promotion in verschiedenen Programmfolgen erfolgen kann und als open-loop oder closed-loop Technik ausgestattet sein kann.

In einer weiteren Ausbildung der Erfindung sind, um den praktischen Einsatz zu verbessern und auszuweiten, als Promoter äthanolische Lösungen, ätherische Öle, chemisch-synthetische Stoffe, die mit äthanolischen oder ätherischen Ölen vergleichbare physikochemische Eigenschaften besitzen, technische oder natürliche Gase eingesetzt, die im Ausgangszustand in unterschiedlichem Aggregatzustand vorliegen können, und die einzeln oder in Form von Kombinationen eingesetzt sind.

In einer weiteren Ausbildung der Erfindung bestehen, um den praktischen Einsatz zu verbessern und auszuweiten, die zur Emissionskontrolle eingesetzten Membranen oder Matrices aus Naturstoffen oder künstlich synthetischen Polymeren, wobei diese auch über Öffnungen zur Emission verfügen können, die mit mechanischen Hilsfmitteln oder mittels Lasertechniken angebracht wurden und deren Durchmesser auch separat dynamisch geregelt werden können.

In einer weiteren Ausbildung der Erfindung sind, um den praktischen Einsatz zu verbessern und auszuweiten, die funktionellen Schichten von chemosensorisch aktivem Wirkstoff und Promoter in einer gemeinsamen Matriacschicht untergebracht.

In einer weiteren Ausbildung der Erfindung liegen, um den praktischen Einsatz zu verbessern und auszuweiten, die räumlichen Dimensionen der Chip-Systeme im Bereich von Zentimetern bis zu Mikrometern.

In einer weiteren Ausbildung der Erfindung sind, um den praktischen Einsatz zu verbessern und auszuweiten, die Chip-Systeme direkt kombiniert mit technischen Vorrichtungen, die die Quantität und Zusammensetzung von Luft beeinflussen oder kontrollieren, insbesondere mit Vorrichtungen zur Beeinflussung von Luftströmung, Lufttemperatur, Luftionisation, Luftfilterung, Luftaromatisierung, Luftfeuchtigkeit, Mischung von Luft mit Flüssigkeiten, Feststoffen oder Gasen.

In einer weiteren Ausbildung der Erfindung werden, um den praktischen Einsatz zu verbessern und auszuweiten, als Materialien wahlweise flexible, dehnbare, transparente, elektrisch leitende, Licht emittierende oder Licht absorbierende, Wärme speichernde oder Wärme emittierende Stoffe, magnetische Materialien, stromleitende Metallfolien, stromleitende Kunststoffe, elektronische Komponenten, poröse natürliche oder synthetische Polymere als Komponenten verarbeitet.

In einer weiteren Ausbildung der Erfindung verfügen, um den praktischen Einsatz zu verbessern und auszuweiten, die Chip-Systeme über Vorrichtungen, mit denen der aktuelle Gehalt oder die biologische Aktivität der chemosensorisch wirkenden Stoffe oder von deren Begleit- oder Abbau-Produkten bestimmt oder angezeigt wird.

In einer weiteren Ausbildung der Erfindung sind, um den praktischen Einsatz zu verbessern und auszuweiten, die Chip-Systeme aus mehreren gleichen oder unterschiedlichen Chip-Systemen zu komplexen Kombinationen erweitert.

In einer weiteren Ausbildung der Erfindung erfolgt, um den praktischen Einsatz zu verbessern und auszuweiten, in den Chip-Systemen ein Einsatz aller natürlichen und chemisch-synthetischen chemsosesenorisch aktiven Stoffe, die zur Beeinflussung von Störungen des zentralen oder vegetativen Nervensystems geeignet sind, insbesondere von Verhaltensstörungen, Stress-Symptomen, Schlafstörungen, Angst- und Aggressionsstörungen, Ess-und Gewichtsstörungen, sexuellen Störungen, Reproduktionsstörungen, Schmerzen, Gefäss- und Kreislaufstörungen, wobei diese als Einzelstoffe oder in Kombinationen vorliegen können.

In einer weiteren Ausbildung der Erfindung erfolgt, um den praktischen Einsatz zu verbessern und auszuweiten, der Einsatz aller Stoffe die im Humanbereich, im tierischen oder pflanzlichen Bereich, sowie allen diesbezüglichen Umweltsystemen in biologischen Regulationsprozessen als chemosensorisch aktive Signalstoffe wirken, einschließlich ihrer natürlichen oder chemisch-synthetischen Analoga, Metabolite, Derivate, Isomere und Antagonisten, als Einzelstoffe oder in Form von Kombinationen.

In einer weiteren Ausbildung der Erfindung erfolgt, um den praktischen Einsatz zu verbessern und auszuweiten, der Einsatz aller chemosensorisch aktiven Stoffe, die in pharmazeutischen und veterinärmedizinischen Erzeugnissen, medizinischen Hilfsmitteln, Produkten für Gesundheits- oder Körperpflege, Kosmetikartikeln oder Parfümartikeln, natürlichen oder künstlichen Nahrungsmitteln und Getränken, diätetischen Erzeugnissen, Pflanzen und landwirtschaftlichen Produkten, biologischen, mikrobiologischen und chemischen Produkten für land-, garten-, forst- und meereswirtschaftliche Zwecke, Mitteln zur Abwehr oder Beseitigung schädlicher Tiere Pflanzen oder Mikroorganismen, Haushalts- Sport- und Gebrauchsartikeln, Verpackungsmaterialien, Bekleidungsstücken enthalten sind, einschließlich aller ihren natürlichen oder chemisch-synthetischen Analoga, Metabolite, Derivate, Isomere oder Antagonisten, als Einzelstoffe oder in Form von Kombinationen.

Vorteile der Erfindung ergeben sich insbesondere dadurch, daß die beschriebenen Chip-Systeme die chemosensorisch aktiven Substanzen, auch über länger vorgegebene Zeiträume reproduzierbar und in vorgegebenen Dosen freisetzen können. Dies kann zudem entweder in Form passiver oder aktiver Emissionsysteme erfolgen. Bei passiven Systemen sind die Emissionsbedingungen durch die entsprechenden Parameter der Komponenten, beispielsweise Porosität der Membran, Art der Promoter, Aggregatzustände, etc. vorgegeben. Aktive Chip-System sind möglich durch interne Implementierung von Steuer- und Kontrollkomponenten, womit die Prozesse auch programmiert erfolgen können. Beispielsweise sind dabei innerhalb eines Chip-Systems unterschiedliche Kontrollvorrichtungen, sowohl für die Emission selbst, wie auch die Prozesse zu deren Promotion untergebracht. Hierbei spielen insbesondere kontrollierte Beeinflussungen thermodynamischer Aktivitäten, somit Steuerungen über Wärmeprozesse, eine wesentliche Rolle. So können beispielsweise physiologisch nicht detektierbare Minima chemosensorischer Stoffen in erwärmten Transportphasen oft noch physiologisch detektierbar werden, d.h. ein chemosensorisch aktives Potential erreichen. Je nach Erfordernis und Anwendungsziel können die Kontrollelemente der Chip-Systeme dabei in alle oder nur in Teilprozesse implementiert sein und dabei entweder als 'open-loop' Systeme oder als rückkoppelnde 'closed-loop' Systeme ausgestaltet sein. Hiermit lassen sich sowohl unterschiedliche Anwendungen ermöglichen und Freisetzungskinetiken auch exakt den neurophysiologischen Bedingungen chemosensorischer rezeptiver Organe anpassen, so beispielsweise Emissionen in bestimmten Zeitintervallen pulsen. Damit lassen sich auch adaptive Gegenreaktionen verringern. Ebenso können in die Chip-Systeme auch verschiedene Meßsysteme zur Aktivitätskontrolle integriert werden, beispielsweise solche, mit denen die Stoffe und deren aktueller Gehalt angezeigt werden kann, woraus sich beispielsweise Aussagen über die aktuelle biologische Aktivität ergeben.

Einzelne Chip-Systeme lassen sich ihrerseits wieder zu komplexen Chip-Gruppen modulmässig erweiternd zusammensetzen. Hierbei sind Anwendungen zahlreicher weiterer Stoffkombinationen möglich. So lassen sich durch solche komplexen Kombinationen Emissions-Muster sowohl qualitativ modifizieren oder auch quantitativ verstärken. Weiterhin können die Chip-Systeme auch mit externen Vorrichtungen kombiniert werden, beispielsweise mit externen Wärmequellen oder mit solchen Vorrichtungen, die die Richtung und Konvektionsstärke der emittierten Stoffe beeinflussen beispielweise an Belüftungsanlagen. Weiterhin lassen sich die Chip-Systmes auch als technische Komponenten in konventionelle Dosiervorrichtungen oder ander technische Vorrichtungen implementieren, z.B. in Spray-Dosierpumpen.

Die Chip-Systeme ermöglichen Anwendungen bei verschiedenen biologischen Fragestellungen. Im Rahmen humantherapeutischer Anwendungen, beispielsweise im Bereich mentaler Stimmungsstörungen. Die Auslösung biologisch chemosensorischer Reaktionen zeigt gegenüber den Effekten einer systemischen Verabreichung üblicher Psychopharmaka signifikante Vorteile. Zentral aktive Psychopharmaka, erzeugen eine erhebliche Anzahl unerwünschter Nebenwirkungen zu denen auch verschiedene Leistungsminderungen gehören. Ihre therapeutischen Effekte sind zudem oft erst Wochen nach Beginn ihrer Einnahme erkennbar, wobei die wesentliche Ursache die systemische Verabreichung ist. Beispielwesie eine orale Anwendung mit Tabletten, beinhaltet die Verteilung des eingenommenen Stoffes im gesamten Körper, wobei dann nur ein kleiner Anteil der verabreichten Substanz, sofern er hierbei die Blut-Hirn Barriere überwinden kann, dann letztlich auch sein Ziel im Zentralen Nervensystem erreicht. Folglich sind solche Effekte sehr unspezifisch und nicht gut kontrollierbar. Demgegenüber bewirken die chemosensorischen Chip-Systeme, daß über die olfaktorische Route mit einem direkter und biologisch wirkenden Mechanismus die chemisch molekularen Informationen der emittierten Stoffe, rasch und selektiv in neuronale Aktivitäten umgesetzt wird. Dies neuronale Vorgehen erzeugt zudem mit weit geringeren Dosen einen schnelleren Wirkeintritt, damit eine höhere Therapiesicherheit. Durch Ausbildung der Chip-Systeme aus und/oder mit flexiblen oder dehnbaren Materialien oder Komponenten, können die Systeme auch pflasterartig und reversibel am Körpers fixiert werden.

Als weiteres Beispiel zur Anwendungsbreite können die Chip-Systeme auch auf körpernahen Gegenständen oder Kleidung angebracht werden, so unter anderem zur Emission chemosensorisch spezifisch wirkender Repellentien gegen stechende oder saugende Insekten, womit die Gefahr der Übertragung von Infektionen, z.B. Malaria, verringert werden kann. Weitere biologische Einsatzmöglichkeiten liegen im Bereich eines umweltfreundlichen landwirtschaftlichen Schutzes gegen Pflanzenschädlinge.

Ein technischer Vorteil ist, daß die Chip-Systeme mit üblichen Produktionsmitteln in größeren Mengen wirtschaftlich, exakt genormt sowie reproduzierbar gefertigt werden können. Weiterhin können sie anwendungsbezogen variabel dimensioniert werden. Die technische Ausgestaltung der Chip-Systeme erlaubt eine Produktion in erheblich unterschiedlichen Dimensionen bis hin zum Mikromaßstab. Einsetzbar für die Massenproduktion sind dabei, sowohl in Teilprozessen, wie auch insgesamt beispielsweise solche Produktionsformen, wie sie bei Drucktechniken oder im Bereich der Mikroelektronik eingesetzt werden. Hierdurch wird auch eine Produktion kleiner Serien, wie auch die nach individuellen Vorgaben, möglich. Insgesamt sind sie daher auch solche technischen Erfordernissse geeignet, wie sie z.B. die Vorschriften für eine Produktion pharmazeutischer oder biotechnischen Produkte verlangt.

Durch ihre Ausgestaltungsmöglichkeiten, unter Einsatz verschiedener technischer Materialien, bieten die Chip-Systeme weiterhin kontrollierte Lagerbedingungen, damit Stabilität und Hygiene auch für empfindliche Stoffe, beispielsweise licht-, temperaturoder oxydationsempfindliche Stoffe. Weiterhin lassen sich sich auch unterschiedlichen Umweltbedingungen anpassen, beispielsweise staubdicht oder wasserdicht gestalten.

Technische prinzipielle Beispiele zum Verfahren sowie den Vorrichtungen für geeignete Chip-Systeme, ohne es auf diese Beispiele beschränken zu wollen, sind nachfolgend erläutert.

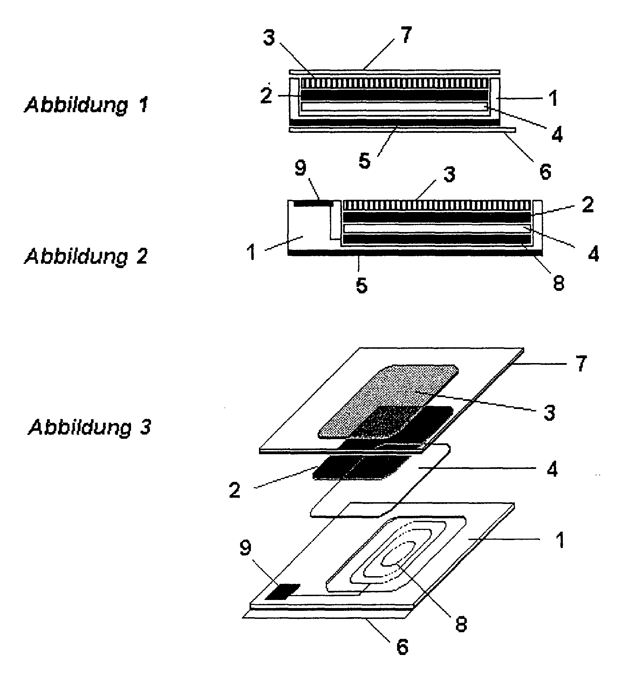

Abbildung 1 zeigt im schematischen Querschnitt den prinzipiellen Aufbau eines Chip-Systems, bei dem sich in einer gemeinsamen Chip-Trägermatrix (1) eine Trägerschicht mit den chemosensorisch aktiven Stoffen befindet (2). Diese ist ihrerseits eingebettet zwischen einer über ihr liegenden, als Membran ausgebildeten, Emissions-Kontrollschicht (3) und einer unter ihr liegenden Komponenten als Promoter-Schicht (4), die beispielsweise Lösungsmittel und/oder Trägervehikel zur Evaporation enthalten kann, und die ihrerseits gegebenenfalls noch mit Reservoirschichten für Promotersubstanz verbunden sein kann. Auf der Unterseite der Chip-Trägermatrix befindet sich eine Adhesiv-Schicht (5), die vor Gebrauch noch mit einer Abdeckschicht (6) versehen ist. Auf der Oberseite der Emissions-Kontrollschicht (3) befindet sich eine entfernbare Deckschicht (7). Nach Entfernung der Deckschicht beginnt durch die Kontrollmembran die Emission der chemosensorischen Stoffe durch Diffusion. Dieser Vorgang wird durch die, in die Stoff-Schicht nachdiffundierenden, Promoter-Substanzen unterstützt und verstärkt.
In Abbildung 2 ist das gleiche Chip-System noch durch eine thermoaktive Massnahme ergänzt, bei der die Promotersubstanz durch eine Heizspirale (8) kontrolliert erwärmt wird. Die Steuerung von Kontrolle, Energieversorgung oder sonstigen Messvorgängen, erfolgt durch in das Chip-System integrierte elektronischen Microchips (9).

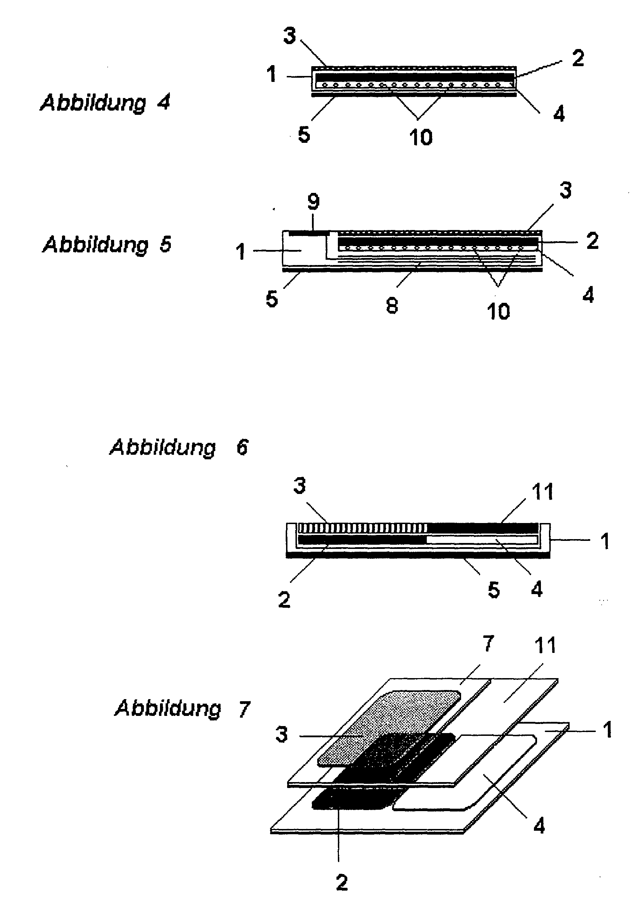

Abbildung 3 zeigt das gleiche System noch einmal schematisch in einem Explosionsschema.
Abbildung 4 zeigt einen prinzipiellen schematischen Aufbau im Querschnitt, bei dem der Aufbau durch Verwendung eines gemeinsamen Matrix-Systems realisiert ist. Die Trägermatrix (1) ist dabei ein für alle operationalen Komponenten gemeinsames Polymer, in das alle Komponenten (2,3,4), ohne ein eigenes Trägersubstrat, schichtweise eingebracht sind. Eine Reservoir-Funktion für die Promoter-Substanzen (4) ist dabei hier in solcher Art realisiert, daß diese in zwei unterschiedlichen Phasen vorliegt. Eine Phase ist hierbei eine schnell diffundierende Form, während die zweite Phase retardiert freisetzt, was beispielsweise in Form retardiert freisetzender Trägerpartikel (10) realisiert sein kann. Die Emissions-Kontrollschicht entspricht hier ebenfalls dem Material des Trägerpolymers und ist auf der Oberseite nur als eine sehr dünne Schicht aufgetragen. Abbildung 5 zeigt den gleichen schematischen Aufbau mit Ergänzung durch eine integrierte aktive Kontrolleinheit (8, 9), wie sie entsprechend schon in den Abbildungen 2 und 3 dargestellt ist.
Abbildung 6 zeigt eine parallele Anbringung von operationalen Schichten. Hierbei sind die Emissions-Kontrollkomponente (3) und die chemosensorische Stoff-Schicht (2) übereinander angebracht, während die Promoter-Schicht (4) in der gleichen Ebene wie die Stoff-Schicht angebracht ist. Über der Promoter-Schicht befindet sich eine spezifische Abdeckung (11), die als ein thermischer Wärmeabsorber ausgestattet sein kann. Damit wird die darunterliegende Promoter-Schicht angewärmt, was zu einem erhöhten Diffusionsdruck zur Stoff-Lage führt. Diese parallele Ausgestaltungsform der chemosensorischen Chip-Systeme, lässt sich somit flacher erstellen. Abbildung 7 zeigt den gleichen Aufbau von Abbildung 6 als ein Explosionsschema.
Die Erfindung betrifft Verfahren und Vorrichtungen von Chip-Systemen zur kontrollierten Emission chemosensorisch wirkender Stoffe, insbesondere zur Erzeugung biologischchemosensorischer Reaktionen. Die Chip-Systeme emittieren kontrolliert wie auch programmierbar Stoffe, die an chemosensorischen Rezeptoren biochemisch Transduktionen für neuronal vermittelte Signale an spezifische Strukturen des Zentralnervensystems auslösen. Das neue Verfahren und seine Vorrichtungen ermöglichen für chemosensorisch aktive Stoffe zahlreiche neue Anwendungen in medizinischen und biologischen Bereichen.

## Patentansprüche

1. Verfahren und Vorrichtungen von Chip-Systemen zur kontrollierten Emission chemosensorisch wirkender Stoffe, insbesondere zur Erzeugung biologisch chemosensorischer Reaktionen, **dadurch gekennzeichnet, daß** die Chips-Systeme als Mehrkomponentensysteme so zusammengesetzt sind, daß die chemosensorisch wirkenden Stoffe in zweifachen Weise kontrolliert als flüchtige Gemische freigesetzt werden, wobei die thermodyamische Diffusionsaktivität der Stoffe durch als Emissions-Promoter dienende Hilfsstoffe und die Emissionsrate der Stoffe durch eine als Diffusionskontrolle dienende Schicht kontrolliert wird, und wobei die Promotion chemisch, physikalisch oder biologisch geregelt werden kann, und wobei sich eine diesem entsprechende Vorrichtung zusammensetzt aus einer Trägerschicht mit den chemosensorisch aktiven Stoffen, einer die Promoter enthaltenden Schicht, die mit weiteren Schichten, die Hilfsvorrichtungen für die Promotion enthalten, verbunden sein kann, einer Emissions-Kontrollschicht, die als Membran oder Polymermatrix ausgebildet ist, sowie einem alle Komponenten einbeziehenden Träger, an dessen äusserer Unterseite sich eine Haftschicht zur Fixierung des Systems und an dessen Oberseite sich eine von der Emissions-Kontrollschicht entfernbare Deckschicht, befinden kann.

2. Verfahren und Vorrichtungen nach Anspruch 1, dadurch kennzeichnet, daß in ihnen die Kontrolle der als Promoter dienenden Hilfsstoffe mechanisch, thermisch, elektrisch, magnetisch, biologisch, chemisch oder biochemisch, oder als eine Kombination solcher Massnahmen erfolgt, wobei die Regelung der Promotion in verschiedenen Programmfolgen erfolgen und als open-loop oder closed loop Technik ausgestattet sein kann.

3. Verfahren und Vorrichtungen nach vorhergehenden Ansprüchen, **dadurch gekennzeichnet, daß** in ihnen als Promoter äthanolische Lösungen, ätherische Öle, Chemisch-synthetische Stoffe, die mit äthanolischen oder ätherischen Ölen vergleichbare physikochemische Eigenschaften besitzen, technische oder natürliche Gase eingesetzt werden, diese im Ausgangszustand in unterschiedlichem
Aggregatzustand vorliegen können, und einzeln oder in Form von Kombinationen eingesetzt sind.

4. Verfahren und Vorrichtungen nach vorhergehenden Ansprüchen, **dadurch gekennzeichnet, daß** die in ihnen zur Emissionskontrolle eingesetzten Membranen oder Matrices aus Naturstoffen oder künstlich synthetischen Polymeren bestehen wobei diese auch über Öffnungen zur Emission verfügen können, die mit mechanischen Hilsfmitteln oder mittels Lasertechniken angebracht wurden und deren Durchmesser auch separat dynamisch geregelt werden können.

5. Verfahren und Vorrichtungen nach vorhergehenden Ansprüchen, **dadurch gekennzeichnet, daß** die funktionellen Schichten von chemosensorisch aktivem Wirkstoff und Promoter in einer gemeinsamen Matrixschicht untergebracht sind.

6. Verfahren und Vorrichtungen nach vorhergehenden Ansprüchen, **dadurch gekennzeichnet, daß** deren räumliche Dimensionen der Chip-Systeme im Bereich von Zentimetern bis zu Mikrometern liegen können.

7. Verfahren und Vorrichtungen nach vorhergehenden Ansprüchen, **dadurch gekennzeichnet, daß** diese direkt kombiniert sind mit technischen Vorrichtungen, die die Quantität und Zusammensetzung von Luft beeinflussen oder kontrollieren, insbesondere Vorrichtungen zur Beeinflussung von Luftströmung, Lufttemperatur, Luftionisation, Luftfilterung, Luftaromatisierung und Luftfeuchtigkeit, Mischung von Luft mit Flüssigkeiten, Feststoffen oder Gasen.

8. Verfahren und Vorrichtungen nach vorhergehenden Ansprüchen, **dadurch gekennzeichnet, daß** in ihnen als Materialien wahlweise flexible, dehnbare, transparente, elektrisch leitende, Licht emittierende oder Licht absorbierende, Wärme speichernde oder Wärme emittierende Stoffe, magnetische Materialien, stromleitende Metallfolien, stromleitende Kunststoffe, elektronische Komponenten, poröse natürliche oder synthetische Polymere als Komponenten verarbeitet sind.

9. Verfahren und Vorrichtungen nach vorhergehenden Ansprüchen, **dadurch gekennzeichnet, daß** sie über Vorrichtungen verfügen, mit denen der aktuelle Gehalt oder die biologische Aktivität an chemosensorisch wirkenden Stoffen oder von deren Begleit- oder Abbau-Produkten bestimmt oder angezeigt wird.

10. Verfahren und Vorrichtungen nach vorhergehenden Ansprüchen, dadurch kennzeichnet, daß diese aus mehreren gleichen oder unterschiedlichen Chip-Systemen zu komplexen Kombinationen erweitert sind.

11. Verfahren und Vorrichtungen nach vorhergehenden Ansprüchen, **dadurch gekennzeichnet, daß** in ihnen alle natürlichen und chemisch-synthetischen chemosensorisch aktiven Stoffe eingesetzt sind, die zur Beeinflussung von Störungen des zentralen oder vegetativen Nervensystems geeignet sind, insbesondere von Verhaltensstörungen, Stress-Symptomen, Schlafstörungen, Angst- und Agressionsstörungen, Ess- und Gewichtsstörungen, sexuellen Störungen, Reproduktionsstörungen, Schmerzen, Gefäss- und Kreislaufstörungen, und als Einzelstoffe oder in Kombinationen vorliegen können.

12. Verfahren und Vorrichtungen nach vorhergehenden Ansprüchen, **dadurch gekennzeichnet, daß** in ihnen alle Stoffe eingesetzt sind, die im Humanbereich, im tierischem oder pflanzlichen Bereich, sowie allen diesbezüglichen Umweltsystemen, in biologischen Regulationsprozessen als chemosensorisch aktive Signalstoffe wirken, einschließlich ihrer natürlichen oder chemisch-synthetischen Analoga, Metabolite, Derivate, Isomeren und Antagonisten, als Einzelstoffe oder in Form von Kombinationen.

13. Verfahren und Vorrichtungen nach vorhergehenden Ansprüchen, **dadurch gekennzeichnet, daß** in ihnen alle chemosensorisch aktiven Stoffe eingesetzt sind, die in pharmazeutischen und veterinärmedizinischen Erzeugnissen, medizinischen Hilfsmitteln, Produkten für Gesundheits- oder Körperpflege, Kosmetikartikeln oder Parfümartikeln, natürlichen oder künstlichen Nahrungsmitteln und Getränken, diätetischen Erzeugnissen, Pflanzen und landwirtschaftlichen Produkten, biologischen, mikrobiologischen und chemischen Produkten für land-, garten-, forst- und meereswirtschaftliche Zwecke, Mitteln zur Abwehr oder Beseitigung schädlicher Tiere Pflanzen oder Mikroorganismen, Haushalts- Sport- und Gebrauchsartikeln, Verpackungsmaterialien, Bekleidungsstücken enthalten sind, einschließlich aller ihren natürlichen oder chemisch-synthetischen Analoga, Metabolite, Derivate, Isomere oder Antagonisten, als Einzelstoffe oder in Form von Kombinationen.
